# EUROPEAN PATENT SPECIFICATION

(11) **EP 3 854 391 B1**
(45) Date of publication and mention of the grant of the patent: **13.03.2024**
(21) Application number: 19861698.9
(22) Date of filing: 20.09.2019
(51) Int. Cl.: A61K 31/41, A61K 31/138, A61K 31/137, A61K 31/4525, A61K 45/06, A61P 25/00

(54) **CARBAMATE COMPOUND AND USE OF FORMULATION COMPRISING SAME IN PREVENTING, ALLEVIATING, OR TREATING ACUTE STRESS DISORDER OR POST-TRAUMATIC STRESS DISORDER**
CARBAMATVERBINDUNG UND VERWENDUNG EINER DIESE UMFASSENDEN FORMULIERUNG ZUR VORBEUGUNG, LINDERUNG ODER BEHANDLUNG VON AKUTER BELASTUNGSSTÖRUNG ODER POSTTRAUMATISCHER BELASTUNGSSTÖRUNG
COMPOSÉ CARBAMATE ET UTILISATION D'UNE FORMULATION COMPRENANT CELUI-CI DANS LA PRÉVENTION, LE SOULAGEMENT OU LE TRAITEMENT D'UN TROUBLE DE STRESS AIGU OU D'UN TROUBLE DE STRESS POST-TRAUMATIQUE

(30) Priority: 21.09.2018 US 201862734403 P
(43) Date of publication of application: 28.07.2021
(73) Proprietor: SK Biopharmaceuticals Co., Ltd., Seongnam-si, Gyeonggi-do 13494 (KR)
(72) Inventor: RYU, Eun Ju, Seongnam-si Gyeonggi-do 13494 (KR); MAENG, Cheol Young, Seongnam-si Gyeonggi-do 13494 (KR); SHIN, Hye Won, Seongnam-si Gyeonggi-do 13494 (KR)
(74) Representative: Vossius & Partner Patentanwälte Rechtsanwälte mbB
(86) International application number: PCT/KR2019/012183
(87) International publication number: WO 2020/060251

(56) References cited:
- WO-A1-2017/200316
- WO-A1-2018/111008
- KR-A- 20030 076 714
- KR-A- 20060 072 127
- KR-A- 20170 131 241
- KR-A- 20180 068 493
- KR-B1- 101 708 433

## Description

### [Technical Field]

The present invention is defined in the appended claims. It relates to use of a carbamate compound of the following
Formula 1 for the purpose of preventing, alleviating or treating acute stress disorder or post-traumatic stress disorder by administering a pharmaceutical composition comprising said carbamate compound: 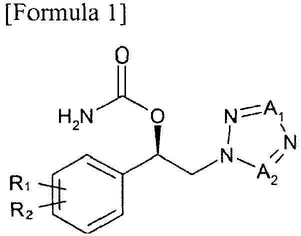 wherein,
R₁, R₂, A₁ and A₂ are as defined herein.

### [Background Art]

"Acute stress disorder" is a psychological condition defined in Diagnostic and Statistical Manual of Mental Disorders, 4th Edition, Text Revision, published by the American Psychiatric Association, Washington, D.C., 2000 ("DSM-IV-TR"). DSM-IV-TR defines "acute stress disorder" as being characterized by symptoms that occur within one month after exposure to an extreme traumatic stressor. DSM-IV-TR lists generally recognized criteria for diagnosing and classifying acute stress disorder.

"Post-traumatic stress disorder (PTSD)" is a psychological condition defined in DSM-IV-TR. DSM-IV-TR defines "post-traumatic stress disorder" as being characterized by continuous re-experiencing of extreme traumatic event(s). DSM-IV-TR lists generally recognized criteria for diagnosing and classifying post-traumatic stress disorder. Extreme traumatic events are events that cause mental trauma which is extreme stresses such as physical abuse, violence, war, life-threatening serious accidents and natural disasters. Most such traumas occur suddenly, causing severe pain for the person experiencing them and reducing their ability to cope with stress. In general, it occurs 5 to 10% of the population, and the prevalence rate of women is about twice as high as that of men.

Symptoms of post-traumatic stress disorder include full or partial amnesia of mental trauma or situation (traumatic events), flashback re-experiencing traumatic events by intrusive memories of that time even though the traumatic events had passed, avoidance of stimuli associated with traumatic events-e.g., activities or places associated with traumatic events, nightmares associated with traumatic events, irritability, hyperarousal (enhanced state of threat sensitivity with the potential for danger), hypervigilance, rage, poor concentration and emotional withdrawal (Nature Reviews Disease Primers volume 1, Article number: 15057 (2015)).

Pharmacological treatment and non-pharmacological treatment can be provided for post-traumatic stress disorder. Only two medications-sertraline and paroxetine, which are anti-depressants having mechanism of action as selective serotonin reuptake inhibitors (SSRIs)-have received approval from the FDA for the treatment of post-traumatic stress disorder. Anti-depressants including these medications are used as the first-line treatment for post-traumatic stress disorder. However, not all patients with PTSD respond to SSRIs and the use of SSRIs is also limited due to adverse effects such as insomnia. In addition to these medications, many medications are used off label for the treatment of post-traumatic stress disorder. Propranolol and prazosin-which have a mechanism of noradrenergic regulation -show partial efficacy in reducing physical symptoms caused by anxiety and alleviating nightmares, respectively. Anti-epileptic drugs-which show efficacy on the kindling model acting on the limbic system of the brain-have been used for alleviating symptoms of anxiety, fear and post-traumatic stress disorder by reducing exaggerated responses to stressors. In addition, various medications such as atypical antipsychotics are also prescribed for each symptom (Journal of Psychiatric Research 36 (2002) 355-367, Curr Psychiatry Rep. 2007 Aug; 9(4): 291-300).

Meanwhile, as a non-pharmacological treatment, behavioral psychotherapy such as cognitive behavior therapy (CBT) or prolonged exposure therapy (PET) is also used to treat post-traumatic stress disorder (Front Behav Neurosci. 2018; 12: 258).

As described above, various pharmacological treatments and non-pharmacological treatments are used to alleviate the symptoms of post-traumatic stress disorder. However, selective serotonin reuptake inhibitors are considered first-line treatment but they are ineffective to all PTSD patients and not tolerated due to adverse effects including insomnia, sexual dysfunction, gastrointestinal symptom, dizziness and headaches. As such, there is a need for new drugs with improved efficacy and safety.

### [Disclosure of the Invention]

### [Problem to be Solved]

The present invention is intended to provide a method for the prevention, alleviation or treatment of acute stress disorder or post-traumatic stress disorder.

The present invention is also intended to provide the use of a carbamate compound of the following Formula 1, or a pharmaceutically acceptable salt, solvate or hydrate thereof, for the prevention, alleviation or treatment of acute stress disorder or post-traumatic stress disorder: 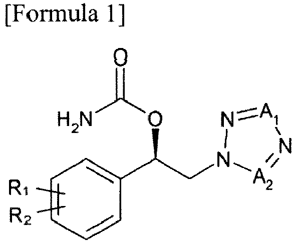 wherein,
R₁, R₂, A₁ and A₂ are as defined herein.

### [Technical Solution to the Problem]

The present invention provides a carbamate compound for use in the prevention, alleviation or
treatment of acute stress disorder or post-traumatic stress disorder, comprising a therapeutically effective amount of a carbamate compound of the following Formula 1, or a pharmaceutically acceptable salt, solvate or hydrate thereof: 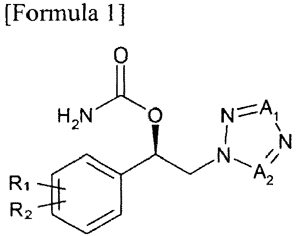 wherein,
R₁ and R₂ are each independently selected from the group consisting of hydrogen, halogen, C₁-C₈ alkyl, halo-C₁-C₈ alkyl, C₁-C₈ thioalkoxy and C₁-C₈ alkoxy; and
one of A₁ and A₂ is CH, and the other is N.

In addition, the present invention provides a pharmaceutical composition for use in the prevention, alleviation or treatment of acute stress disorder or post-traumatic stress disorder, comprising a therapeutically effective amount of the carbamate compounds of the above Formula 1, or a pharmaceutically acceptable salt, solvate or hydrate thereof, and further one or more of a pharmaceutically acceptable carrier.

In addition, the present invention provides the claimed compounds for use in a method for preventing, alleviating or
treating acute stress disorder or post-traumatic stress disorder, in a subject, comprising administering to the subject a therapeutically effective amount of the carbamate compounds of the above Formula 1, or a pharmaceutically acceptable salt, solvate or hydrate thereof.

In addition, the present invention provides the carbamate compounds of the above Formula 1, or a pharmaceutically acceptable salt, solvate or hydrate thereof for use in the prevention, alleviation or treatment of acute stress disorder or post-traumatic stress disorder, or for the improvement of symptoms associated therewith.

According to one embodiment of the present invention, in the above Formula 1, R₁ and R₂ are each independently selected from the group consisting of hydrogen, halogen and C₁-C₈ alkyl.

In one embodiment of the present invention, the halo C₁-C₈ alkyl is perfluoroalkyl.

According to another embodiment of the present invention, the carbamate compound of the above Formula 1 is carbamic acid (R)-1-(2-chlorophenyl)-2-tetrazol-2-yl-ethyl ester of the following Formula 2:

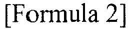

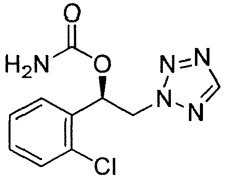

A person having ordinary skill in the art of synthesis of compounds could have easily prepared the carbamate compounds of the above Formulas 1 and 2 using known compounds or compounds which can be easily prepared therefrom. Specifically, methods for preparing the compounds of the above Formula 1 are described in detail in International Publication Nos. WO 2006/112685 A1, WO 2010/150946 A1 and WO 2011/046380 A2.

The compounds of the above Formula 1 can be chemically synthesized by any of the methods described in the above documents, but the methods are merely exemplary ones, and the order of the unit operation and the like may be selectively changed if necessary.

The carbamate compounds of the above Formula 1 can be used for the prevention, alleviation or treatment of acute stress disorder or post-traumatic stress disorder.

The dosage of the carbamate compounds of Formula 1 for the prevention, alleviation or treatment of the above diseases may typically vary depending on the severity of the disease, the body weight and the metabolic status of the subject. A "therapeutically effective amount" for an individual patient refers to an amount of the active compound sufficient to achieve the above pharmacological effect, i.e., the therapeutic effect as described above. The therapeutically effective amount of the compounds of the present invention is 50 to 500 mg, 50 to 400 mg, 50 to 300 mg, 100 to 400 mg, 100 to 300 mg, 50 to 200 mg, or 100 to 200 mg, based on the free form and once-daily administration to humans. The therapeutically effective amount is preferably 50 to 300 mg, more preferably 50 to 200 mg.

The compounds of the present invention may be administered by any conventional method used for administration of a therapeutic agent, such as oral, parenteral, intravenous, intramuscular, subcutaneous or rectal administration.

The medicament or pharmaceutical composition according to one embodiment of the present invention may comprise a therapeutically effective amount of a compound selected from the group consisting of the carbamate compounds of the present invention, their pharmaceutically acceptable salts, solvates, hydrates and combinations thereof.

Examples of the pharmaceutically acceptable salts of the carbamate compounds of the above Formula 1 include independently, acetate, benzenesulfonate, benzoate, bitartrate, calcium acetate, camsylate, carbonate, citrate, edetate, edisylate, estolate, esylate, fumarate, gluceptate, gluconate, glutamate, glycoloyl arsanilate, hexylresorcinate, hydravamine, hydrobromide, hydrochloride, hydrogencarbonate, hydroxynaphthoate, iodide, isethionate, lactate, lactobionate, malate, maleate, mandelate, mesylate, methylnitrate, methylsulfate, mucate, napsylate, nitrate, pamoate (embonate), pantothenate, phosphate/diphosphate, polygalacturonate, salicylate, stearate, subacetate, succinate or hemi-succinate, sulfate or hemi-sulfate, tannate, tartrate, oxalate or hemi-tartrate, teoclate, triethiodide, benzathine, chloroprocaine, choline, diethanolamine, ethylenediamine, meglumine, procaine, aluminum, ammonium, tetramethylammonium, calcium, lithium, magnesium, potassium, sodium and zinc.

The medicament or pharmaceutical composition according to one embodiment of the present invention may be administered orally or parenterally. The parenteral administration may include intravenous injection, subcutaneous injection, intramuscular injection, intraperitoneal injection, endothelial administration, topical administration, intranasal administration, intravaginal administration, intrapulmonary administration, rectal administration and the like. In the case of oral administration, the pharmaceutical composition according to one embodiment of the present invention may be formulated as a plain tablet (uncoated tablet) or such that the active agent is coated or it is protected against degradation in the stomach. In addition, the composition can be administered by any device capable of transferring the active substance to a target cell. The route of administration may vary depending upon the general condition and age of the subject to be treated, the nature of the treatment condition and the active ingredient selected.

A suitable dosage of the medicament or pharmaceutical composition according to one embodiment of the present invention may vary depending on factors such as the formulation method, administration method, age, body weight and gender of patients, pathological condition, diet, administration time, administration route, excretion rate and reaction sensitivity, and doctors having ordinary skill can easily determine and prescribe dosages that are effective for the desired treatment or prophylaxis. The pharmaceutical composition according to one embodiment may be administered in one or more doses, for example, one to four times per day. The pharmaceutical composition according to one embodiment may contain the compounds of Formula 1 in the amount of 50 to 500 mg, 50 to 400 mg, 50 to 300 mg, 100 to 400 mg, 100 to 300 mg, 50 to 200 mg, or 100 to 200 mg, preferably 50 to 300 mg, more preferably 50 to 200 mg, based on the free form. The medicament or pharmaceutical composition according to one embodiment of the present invention may be formulated using a pharmaceutically acceptable carrier and/or excipient according to a method that a person having ordinary skill in the art could easily carry out, thereby to be prepared in a unit dose form or to be contained in a multi-dose container. The above formulation may be a solution in oil or an aqueous medium, a suspension or an emulsion (emulsified solution), an extract, a powder, granules, a tablet, or a capsule, and may further include a dispersing or stabilizing agent. In addition, the pharmaceutical composition may be administered in the form of suppositories, sprays, ointments, creams, gels, inhalants or skin patches. The pharmaceutical composition may also be prepared for mammalian administration, more preferably for human administration.

Pharmaceutically acceptable carriers may be solid or liquid, and may be one or more selected from fillers, antioxidants, buffers, bacteriostats, dispersants, adsorbents, surfactants, binders, preservatives, disintegrants, sweeteners, flavors, glidants, release-controlling agents, wetting agents, stabilizers, suspending agents, and lubricants. In addition, the pharmaceutically acceptable carriers may be selected from saline, sterile water, Ringer's solution, buffered saline, dextrose solution, maltodextrin solution, glycerol, ethanol and mixtures thereof.

In one embodiment, suitable fillers include, but are not limited to, sugar (e.g., dextrose, sucrose, maltose and lactose), starch (e.g., corn starch), sugar alcohol (e.g., mannitol, sorbitol, maltitol, erythritol and xylitol), starch hydrolysate (e.g., dextrin and maltodextrin), cellulose or cellulose derivatives (e.g., microcrystalline cellulose) or mixtures thereof.

In one embodiment, suitable binders include, but are not limited to, povidone, copovidone, methylcellulose, hydroxypropylmethylcellulose, hydroxypropylcellulose, hydroxyethylcellulose, gelatin, gum, sucrose, starch or mixtures thereof.

In one embodiment, suitable preservatives include, but are not limited to, benzoic acid, sodium benzoate, benzyl alcohol, butylated hydroxyanisole, butylated hydroxytoluene, chlorbutol, gallate, hydroxybenzoate, EDTA or mixtures thereof.

In one embodiment, suitable disintegrants include, but are not limited to, sodium starch glycolate, cross-linked polyvinylpyrrolidone, cross-linked carboxymethylcellulose, starch, microcrystalline cellulose or mixtures thereof.

In one embodiment, suitable sweeteners include, but are not limited to, sucralose, saccharin, sodium saccharin, potassium saccharin, calcium saccharin, acesulfame potassium or sodium cyclamate, mannitol, fructose, sucrose, maltose or mixtures thereof.

In one embodiment, suitable glidants include, but are not limited to, silica, colloidal silicon dioxide, talc and the like.

In one embodiment, suitable lubricants include, but are not limited to, long chain fatty acids and salts thereof, such as magnesium stearate and stearic acid, talc, glyceride wax or mixtures thereof.

According to still another embodiment of the present invention, there is provided a combination for the prevention, alleviation or treatment of acute stress disorder or post-traumatic stress disorder, comprising (a) a therapeutically effective amount of a carbamate compound of the following Formula 1, or a pharmaceutically acceptable salt, solvate or hydrate thereof; and (b) a selective serotonergic reuptake inhibitor (SSRI): 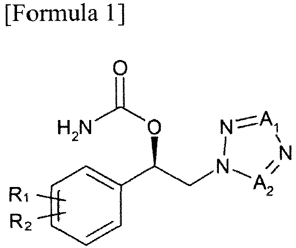 wherein,
R₁ and R₂ are each independently selected from the group consisting of hydrogen, halogen, Ci-Cs alkyl, halo-C₁-C₈ alkyl, C₁-C₈ thioalkoxy and C₁-C₈ alkoxy; and
one of A₁ and A₂ is CH, and the other is N.

According to still another embodiment of the present invention, the selective serotonergic reuptake inhibitor may be one or more selected from the group consisting of fluoxetine, sertraline, paroxetine and a pharmaceutically acceptable salt thereof, but is not limited thereto.

According to still another embodiment of the present invention, in the combination, the weight ratio (a:b) of the ingredient (a) and the ingredient (b) may be within the scope of 1,000:1 to 1:1,000. According to still another embodiment of the present invention, in the combination, the weight ratio (a:b) of the ingredient (a) and the ingredient (b) may be within the scope of 100:1 to 1:100.

According to still another embodiment of the present invention, the combination may comprise the compound of Formula 1 in an amount of 12.5 to 500 mg, based on the free form.

According to still another embodiment of the present invention, the selective serotonergic reuptake inhibitor may be fluoxetine or a pharmaceutically acceptable salt thereof. According to still another embodiment of the present invention, the selective serotonergic reuptake inhibitor may be fluoxetine hydrochloride.

According to still another embodiment of the present invention, the combination may comprise fluoxetine in an amount of 10 to 60 mg, based on the free form.

According to still another embodiment of the present invention, there is provided a carbamate compound for use in
the prevention, alleviation or treatment of symptom of post-traumatic stress disorder, comprising a therapeutically effective amount of a carbamate compound of the following Formula 1, or a pharmaceutically acceptable salt, solvate or hydrate thereof: 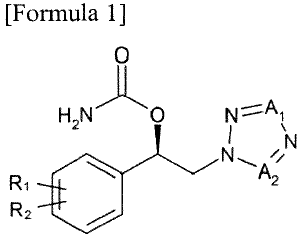 wherein,
R₁ and R₂ are each independently selected from the group consisting of hydrogen, halogen, C₁-C₈ alkyl, halo-Ci-Cs alkyl, C₁-C₈ thioalkoxy and C₁-C₈ alkoxy; and
one of A₁ and A₂ is CH, and the other is N.

According to still another embodiment of the present invention, the symptom of post-traumatic stress disorder is one or more selected from the group consisting of full or partial amnesia of traumatic event, flashback in a patient re-experiencing traumatic event or avoidance of stimulus associated with traumatic event, nightmare, irritability, hyperarousal, hypervigilance, rage, poor concentration and emotional withdrawal.

As used herein, the terms "prevent," "preventing" and "prevention" refer to reducing or eliminating the likelihood of a disease.

As used herein, the terms "alleviate," "alleviating" and "alleviation" refer to ameliorating a disease and/or its accompanying symptoms altogether or in part.

As used herein, the terms "treat," "treating" and "treatment" refer to eliminating a disease and/or its accompanying symptoms altogether or in part.

As used herein, the term "subject" refers to an animal that is the object of therapy, observation or experiment, preferably a mammal (such as primates (e.g., a human), cattle, sheep, goats, horses, dogs, cats, rabbits, rats, mice, etc.), most preferably a human.

As used herein, the term "therapeutically effective amount" refers to the amount of active compound or pharmaceutical formulation that elicits a biological or medical response in the system, animal or human, including alleviation of the symptoms of the disease or disorder to be treated, wherein said amount is sought by a researcher, veterinarian, doctor (physician) or other clinician.

As used herein, the term "composition" encompasses a product that contains a specified amount of a particular ingredient and any product that results directly or indirectly from a combination of specified amounts of particular ingredients.

### [Effect of the Invention]

The medicament and the combination according to the present invention can effectively prevent, alleviate and treat acute stress disorder or post-traumatic stress disorder.

### [Brief Description of the Drawings]

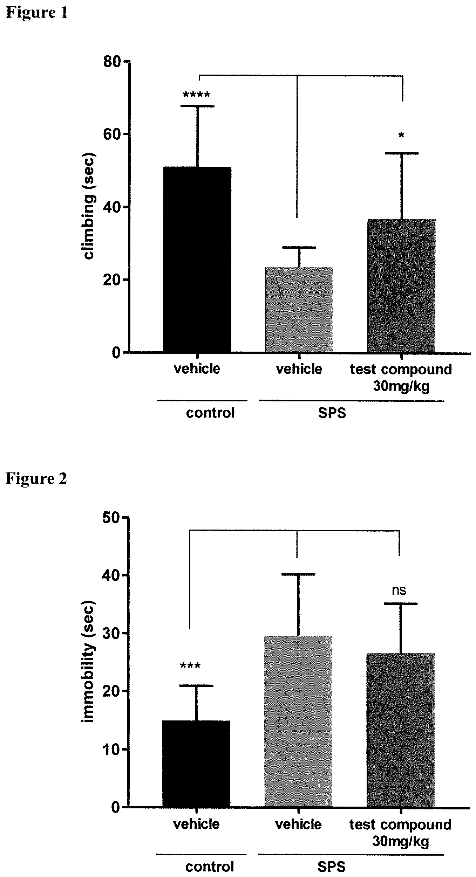

Figure 1 shows the comparison of climbing behavior by carrying out forced swimming test (FST) in rats in which post-traumatic stress disorder was not induced, rats in which post-traumatic stress disorder was induced by single prolonged stress (SPS) and rats in which post-traumatic stress disorder was induced by SPS and carbamic acid (R)-1-(2-chlorophenyl)-2-tetrazol-2-yl-ethyl ester (Test Compound) prepared in the Preparation Example was then administered (left, middle and right, respectively).
Figure 2 shows the comparison of immobility time by carrying out forced swimming test in rats in which post-traumatic stress disorder was not induced, rats in which post-traumatic stress disorder was induced by SPS and rats in which post-traumatic stress disorder was induced by SPS and carbamic acid (R)-1-(2-chlorophenyl)-2-tetrazol-2-yl-ethyl ester (Test Compound) prepared in the Preparation Example was then administered (left, middle and right, respectively).

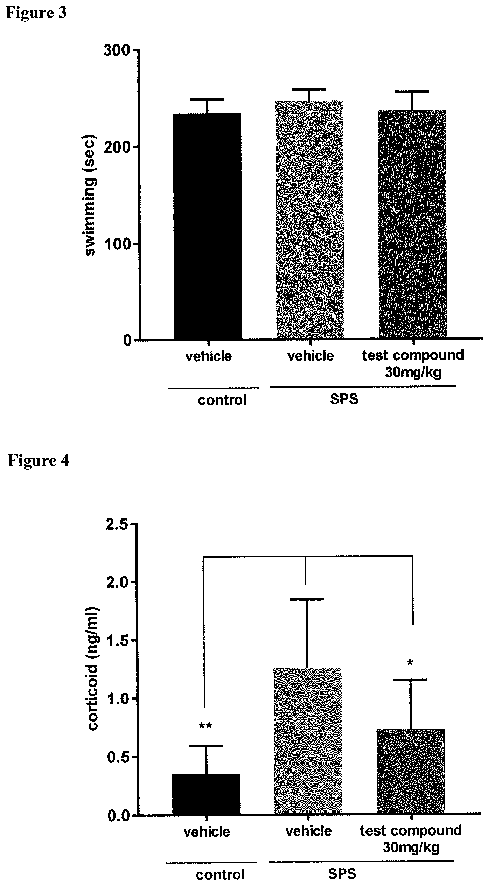

Figure 3 shows the comparison of swimming time by carrying out forced swimming test in rats in which post-traumatic stress disorder was not induced, rats in which post-traumatic stress disorder was induced by SPS and rats in which post-traumatic stress disorder was induced by SPS and carbamic acid (R)-1-(2-chlorophenyl)-2-tetrazol-2-yl-ethyl ester (Test Compound) prepared in the Preparation Example was then administered (left, middle and right, respectively).
Figure 4 shows the comparison of corticoid concentrations in the blood of rats in which post-traumatic stress disorder was not induced, rats in which post-traumatic stress disorder was induced by SPS and rats in which post-traumatic stress disorder was induced by SPS and carbamic acid (R)-1-(2-chlorophenyl)-2-tetrazol-2-yl-ethyl ester (Test Compound) prepared in the Preparation Example was then administered.

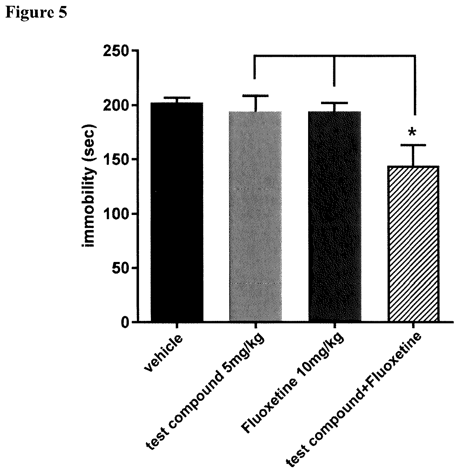

Figure 5 shows the synergistic effect on immobility time in mice in which carbamic acid (R)-1-(2-chlorophenyl)-2-tetrazol-2-yl-ethyl ester (Test Compound) prepared in the Preparation Example was administered together with fluoxetine hydrochloride.

### [Specific Embodiments to Carry Out the Invention]

Hereinafter, the present invention will be explained in more detail through working examples. However, the following working examples are only intended to illustrate one or embodiments. more

### Preparation Example: Synthesis of carbamic acid (R)-1-(2-chlorophenyl)-2-tetrazol-2-yl-ethyl ester

Carbamic acid (R)-1-(2-chlorophenyl)-2-tetrazol-2-yl-ethyl ester (hereinafter referred to as "Test Compound") was prepared according to the method described in Preparation Example 50 of International Publication No. WO 2010/150946.

### Example 1: Forced Swimming Test

### Experimental animals

Adult male rats (Sprague-Dawley, 200 to 230 g) were used. The experimental animals were maintained at a light-and-darkness cycle of 12 hours (illuminated from 7 pm to 7 am), a temperature of 22 to 25°C, a relative humidity of 40 to 60%, and free access to water and food. The animals were randomly divided into three groups as follows:
- Fourteen (14) rats as a control group, orally administered with saline as a vehicle at a dose volume of 3 ml/kg (Group 1)
- Twelve (12) rats in which post-traumatic stress disorder was induced, and saline as a vehicle was then orally administered at a dose volume of 3 ml/kg (Group 2)
- Twelve (12) rats in which post-traumatic stress disorder was induced, and Test Compound was then orally administered at a dose of 30 mg/kg (3 ml/kg) (Group 3)

### Induction of post-traumatic stress disorder

To establish optimal post-traumatic stress disorder, animal model induced by single prolonged stress (SPS). The animals expressing post-traumatic stress disorder was established by exposure to stress under various conditions (2 hours of restraint stress, 20 minutes of forced swimming, 15 minutes of rest, exposure to ether vapor).

Post-traumatic stress disorder was induced in Group 2 and Group 3 as described above. Following SPS stressors, rats were housed in their cages alone and left undisturbed for seven days to induce post-traumatic stress. All experimental animals were measured daily for changes in mortality and body weight to determine whether post-traumatic stress disorder was well induced.

### Administration

For 15 days from the day following the exposure to single prolonged stress, Group 2 was orally administered with saline as a vehicle and Group 3 with 30 mg/kg (3 ml/kg) of Test Compound.

Meanwhile, Group 1-in which post-traumatic stress disorder was not induced-was orally administered with saline at the same time when oral administration was started in Group 2 and Group 3.

### Forced swimming test

To carry out a forced swimming test in the rats of Groups 1 to 3, water of 25°C was filled to a height of 30 cm in a transparent cylinder (20 cm in diameter × 50 cm in height). The forced swimming test consisted of two sessions. On the first day, rats were acclimated in the cylinder filled with water for 15 minutes. After 24 hours, rats were inserted into the cylinder for 5 minutes and the escape behavior of rats was recorded and measured using a video camera. Rats' behaviors were divided into climbing, immobility and swimming behaviors. Climbing behavior measured the time to climb upward, immobility behavior measured the time to not escape, and swimming behavior measured the time to move horizontally in the cylinder. The results are represented in Figures 1 to 3, respectively.

### Statistics

The efficacy of the compound was expressed as mean ± standard error of mean (SEM), and statistical significance was recognized when data had a difference of p <0.05. Statistical analysis was carried out by using one-way ANOVA in Prism 7.04.

From the above results, it was confirmed that Test Compound can be used as a medication for the prevention, alleviation or treatment of post-traumatic stress disorder by showing efficacy on alleviating symptoms associated with post-traumatic stress disorder.

### Example 2: Corticoid Change

### Experimental animals

Adult male rats (Sprague-Dawley, 200 to 230 g) were used. The experimental animals were maintained at a light-and-darkness cycle of 12 hours (illuminated from 7 pm to 7 am), a temperature of 22 to 25°C, a relative humidity of 40 to 60%, and free access to water and food. The animals were randomly divided into three groups as follows:
- Eight (8) rats as a control group, orally administered with saline as a vehicle at a dose volume of 3 ml/kg (Group 1)
- Seven (7) rats in which post-traumatic stress disorder was induced, and saline as a vehicle was then orally administered at a dose volume of 3 ml/kg (Group 2)
- Seven (7) rats in which post-traumatic stress disorder was induced, and Test Compound was then orally administered at a dose of 30 mg/kg (3 ml/kg) (Group 3)

### Induction of post-traumatic stress disorder

To establish optimal post-traumatic stress disorder, animal model induced by single prolonged stress (SPS). The animals expressing post-traumatic stress disorder was established by exposure to stress under various conditions (2 hours of restraint stress, 20 minutes of forced swimming, 15 minutes of rest, exposure to ether vapor).

Post-traumatic stress disorder was induced in Group 2 and Group 3 as described above. Following SPS stressors, rats were housed in their cages alone and left undisturbed for seven days to induce post-traumatic stress. All experimental animals were measured daily for changes in mortality and body weight to determine whether post-traumatic stress disorder was well induced.

### Administration

For 15 days from the day following the exposure to single prolonged stress, Group 2 was orally administered with saline as a vehicle and Group 3 with 30 mg/kg (3 ml/kg) of Test Compound.

Meanwhile, Group 1-in which post-traumatic stress disorder was not induced-was orally administered with saline at the same time when oral administration was started in Group 2 and Group 3.

### Measurement of corticoid in blood

Rats were sacrificed rapidly by decapitation on the day after the forced swimming test, and blood was quickly collected via the abdominal aorta. The blood sample was centrifuged at 4,000 g for 10 minutes, and serum was collected and stored at -20°C. The corticoid concentration was measured by enzyme-linked immunoassay (ELISA) using a Novus Biologicals Corticosterone kit. The results were measured by means of an ELISA reader (MultiRead 400) to calculate the concentration of corticoid.

### Statistics

The efficacy of the compound was expressed as mean ± standard error of mean (SEM), and statistical significance was recognized when data had a difference of p <0.05. Statistical analysis was carried out by using one-way ANOVA in Prism 7.04.

As can be seen from Figure 4, when Test Compound was orally administered at a dose of 30 mg/kg, there was a statistically significant reduction in the blood corticoid concentration, as compared with the control group in which saline as a vehicle was orally administered to the post-traumatic stress disorder-induced rats. Because a subject having post-traumatic stress disorder is in a state of increasing blood corticoid concentration, it was confirmed that Test Compound can be used as a medication for the prevention, alleviation or treatment of post-traumatic stress disorder in view that Test Compound shows the effect on lowering the increased blood corticoid concentration.

### Example 3: Forced Swimming Test by Using Combination of Test Compound and Selective Serotonergic Reuptake Inhibitor

### Experimental animals

Adult mice (CD-1) were used. The experimental animals were maintained at a light-and-darkness cycle of 12 hours (illuminated from 7 pm to 7 am), a temperature of 22 to 25°C, a relative humidity of 40 to 60%, and free access to water and food. The animals were randomly divided into four groups as follows:
- Eight (8) mice as a control group, administered with intraperitoneal injection of saline as a vehicle at a dose volume of 10 ml/kg (Group 1)
- Eight (8) mice as an experimental group, administered with intraperitoneal injection of Test Compound at a dose of 5 mg/kg (10 ml/kg) (Group 2)
- Eight (8) mice as an experimental group, administered with intraperitoneal injection of fluoxetine hydrochloride at a dose of 10 mg/kg (10 ml/kg) (Group 3)
- Eight (8) mice as an experimental group, administered with intraperitoneal injection of Test Compound at a dose of 5 mg/kg (10 ml/kg) and fluoxetine hydrochloride at a dose of 10 mg/kg (10 ml/kg) (Group 4)

### Forced swimming test

To perform a forced swimming test in the mice of Groups 1 to 4, water of 25°C was filled to a height of 20 cm in a transparent cylinder. 30 Min after injection, mice were acclimated in water for 2 minutes, and the duration of despair behavior that did not move was measured for 4 minutes using a timer. The results are represented in Figure 5.

### Statistics

The effect of the compound was expressed as mean ± standard error of mean (SEM), and statistical significance was recognized when data had a difference of p <0.05. Statistical analysis was carried out by using one-way ANOVA in Prism 7.04.

From the above results, a combination of Test Compound and selective serotonergic reuptake inhibitor (fluoxetine)-which are at an ineffective dose, respectively-was shown to be synergistic in a forced swimming test. With this, it was confirmed that co-administration of Test Compound and selective serotonergic reuptake inhibitor-which has been used as the first-line treatment for post-traumatic stress disorder-may have synergistic efficacy.

## Claims

1. A medicament for use in the prevention, alleviation or treatment of acute stress disorder or post-traumatic stress disorder, comprising a therapeutically effective amount of a carbamate compound of the following Formula 1, or a pharmaceutically acceptable salt, solvate or hydrate thereof: 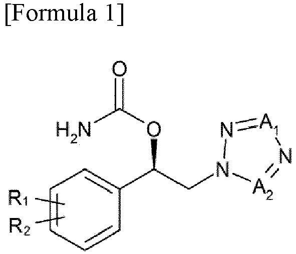 wherein,
R₁ and R₂ are each independently selected from the group consisting of hydrogen, halogen, C₁-C₈ alkyl, halo-C₁-C₈ alkyl, C₁-C₈ thioalkoxy and C₁-C₈ alkoxy; and
one of A₁ and A₂ is CH, and the other is N.

2. The medicament for use according to Claim 1, wherein R₁ and R₂ are each independently selected from the group consisting of hydrogen, halogen and Ci-Cs alkyl.

3. The medicament for use according to Claim 1, wherein the carbamate compound of Formula 1 is carbamic acid (R)-1-(2-chlorophenyl)-2-tetrazol-2-yl-ethyl ester of the following Formula 2:

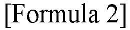

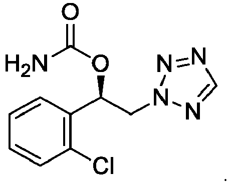

4. A combination for use in the prevention, alleviation or treatment of acute stress disorder or post-traumatic stress disorder, comprising:
(a) a therapeutically effective amount of a carbamate compound of the following Formula 1, or a pharmaceutically acceptable salt, solvate or hydrate thereof; and
(b) a selective serotonergic reuptake inhibitor (SSRI): 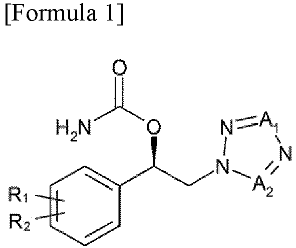 wherein,
R₁ and R₂ are each independently selected from the group consisting of hydrogen, halogen, C₁-C₈ alkyl, halo-C₁-C₈ alkyl, C₁-C₈ thioalkoxy and C₁-C₈ alkoxy; and
one of A₁ and A₂ is CH, and the other is N.

5. The combination for use according to Claim 4, wherein the selective serotonergic reuptake inhibitor is one or more selected from the group consisting of fluoxetine, sertraline, paroxetine and a pharmaceutically acceptable salt thereof.

6. The combination for use according to Claim 4, wherein the weight ratio of a:b is from 1,000: 1 to 1: 1,000.

7. The combination for use according to Claim 6, wherein the weight ratio of a:b is from 100:1 to 1:100.

8. The combination for use according to Claim 4, which comprises the compound of Formula 1 in an amount of 12.5 mg to 500 mg based on the free form.

9. The combination for use according to Claim 5, wherein the selective serotonergic reuptake inhibitor is fluoxetine or a pharmaceutically acceptable salt thereof.

10. The combination for use according to Claim 9, wherein the selective serotonergic reuptake inhibitor is fluoxetine hydrochloride.

11. The combination for use according to Claim 9, which comprises fluoxetine in an amount of 10 to 60 mg based on the free form.

12. A medicament for use in the prevention, alleviation or treatment of symptom of post-traumatic stress disorder, comprising a therapeutically effective amount of a carbamate compound of the following Formula 1, or a pharmaceutically acceptable salt, solvate or hydrate thereof; and further one or more of a pharmaceutically acceptable carrier: 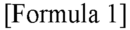 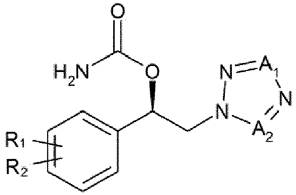 wherein,
R₁ and R₂ are each independently selected from the group consisting of hydrogen, halogen, C₁-C₈ alkyl, halo-C₁-C₈ alkyl, C₁-C₈ thioalkoxy and C₁-C₈ alkoxy; and
one of A₁ and A₂ is CH, and the other is N.

13. The medicament for use according to Claim 12, wherein the symptom of post-traumatic stress disorder is one or more selected from the group consisting of full or partial amnesia of traumatic event, flashback in a patient re-experiencing traumatic event or avoidance of stimulus associated with traumatic event, nightmare, irritability, hyperarousal, hypervigilance, rage, poor concentration and emotional withdrawal.

## Patentansprüche

1. Ein Medikament zur Verwendung bei der Prävention, Linderung oder Behandlung von akuter Belastungsstörung oder posttraumatischer Belastungsstörung, umfassend eine therapeutisch wirksame Menge einer Carbamatverbindung der folgenden Formel 1 oder eines pharmazeutisch verträglichen Salzes, Solvats oder Hydrats davon: 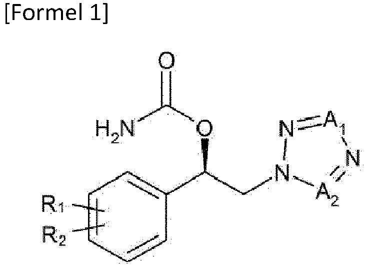 wobei
R₁ und R₂ jeweils unabhängig ausgewählt sind aus der Gruppe bestehend aus Wasserstoff, Halogen, C₁-C₈-Alkyl, Halogen-C₁-C₈-alkyl, C₁-C₈-Thioalkoxy und C₁-C₈-Alkoxy; und
eines von A₁ und A₂ gleich CH ist und das andere N ist.

2. Das Medikament zur Verwendung nach Anspruch 1, wobei R₁ und R₂ jeweils unabhängig ausgewählt sind aus der Gruppe bestehend aus Wasserstoff, Halogen und C₁-C₈-Alkyl.

3. Das Medikament zur Verwendung nach Anspruch 1, wobei die Carbamatverbindung der Formel 1 Carbaminsäure-(R)-1-(2-chlorphenyl)-2-tetrazol-2-yl-ethylester der folgenden Formel 2 ist:

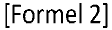

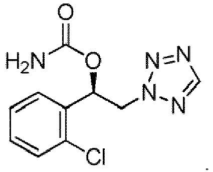

4. Eine Kombination zur Verwendung bei der Prävention, Linderung oder Behandlung von akuter Belastungsstörung oder posttraumatischer Belastungsstörung, umfassend:
(a) eine therapeutisch wirksame Menge einer Carbamatverbindung der folgenden Formel 1 oder eines pharmazeutisch verträglichen Salzes, Solvats oder Hydrats davon; und
(b) einen selektiven Serotonin-Wiederaufnahmehemmer (SSRI): 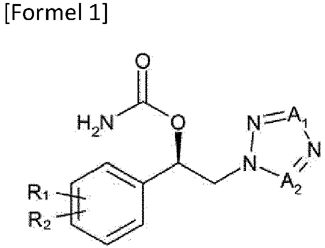 wobei
R₁ und R₂ jeweils unabhängig ausgewählt sind aus der Gruppe bestehend aus Wasserstoff, Halogen, C₁-C₈-Alkyl, Halogen-C₁-C₈-alkyl, C₁-C₈-Thioalkoxy und C₁-C₈-Alkoxy; und
eines von A₁ und A₂ gleich CH ist und das andere N ist.

5. Die Kombination zur Verwendung nach Anspruch 4, wobei der selektive Serotonin-Wiederaufnahmehemmer einer oder mehrere, ausgewählt aus der Gruppe bestehend aus Fluoxetin, Sertralin, Paroxetin und einem pharmazeutisch verträglichen Salz davon, ist.

6. Die Kombination zur Verwendung nach Anspruch 4, wobei das Gewichtsverhältnis von a:b 1.000:1 bis 1:1.000 beträgt.

7. Die Kombination zur Verwendung nach Anspruch 6, wobei das Gewichtsverhältnis von a:b 100:1 bis 1:100 beträgt.

8. Die Kombination zur Verwendung nach Anspruch 4, welche die Verbindung der Formel 1 in einer Menge von 12,5 mg bis 500 mg, bezogen auf die freie Form, umfasst.

9. Die Kombination zur Verwendung nach Anspruch 5, wobei der selektive Serotonin-Wiederaufnahmehemmer Fluoxetin oder ein pharmazeutisch verträgliches Salz davon ist.

10. Die Kombination zur Verwendung nach Anspruch 9, wobei der selektive Serotonin-Wiederaufnahmehemmer Fluoxetin-Hydrochlorid ist.

11. Die Kombination zur Verwendung nach Anspruch 9, welche Fluoxetin in einer Menge von 10 bis 60 mg, bezogen auf die freie Form, umfasst.

12. Ein Medikament zur Verwendung bei der Prävention, Linderung oder Behandlung von Symptomen posttraumatischer Belastungsstörung, umfassend eine therapeutisch wirksame Menge einer Carbamatverbindung der folgenden Formel 1 oder eines pharmazeutisch verträglichen Salzes, Solvats oder Hydrats davon, und weiter einen oder mehrere pharmazeutisch verträgliche Träger: 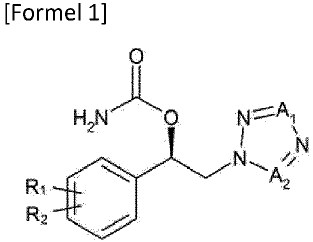 wobei
R₁ und R₂ jeweils unabhängig ausgewählt sind aus der Gruppe bestehend aus Wasserstoff, Halogen, C₁-C₈-Alkyl, Halogen-C₁-C₈-alkyl, C₁-C₈-Thioalkoxy und C₁-C₈-Alkoxy; und
eines von A₁ und A₂ gleich CH ist und das andere N ist.

13. Das Medikament zur Verwendung nach Anspruch 12, wobei das Symptom posttraumatischer Belastungsstörung eines oder mehrere, ausgewählt aus der Gruppe bestehend aus vollständiger oder teilweiser Amnesie eines traumatischen Ereignisses, Flashback bei einem Patienten, der ein traumatisches Erlebnis erneut durchlebt, oder Vermeidung von Auslösern, die mit einem traumatischen Ereignis in Zusammenhang stehen, Albtraum, Reizbarkeit, Übererregbarkeit, Überwachheit, Zorn, Konzentrationsschwäche und emotionalem Rückzug, ist.

## Revendications

1. Médicament pour son utilisation dans la prévention, le soulagement ou le traitement d'un trouble de stress aigu ou d'un trouble de stress post-traumatique, comprenant une quantité thérapeutiquement efficace d'un composé carbamate de formule 1 qui suit, ou d'un sel, solvate ou hydrate pharmaceutiquement acceptable de celui-ci : 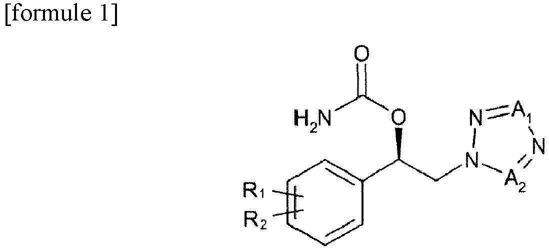 dans lequel
chacun de R₁ et R₂ est indépendamment choisi dans le groupe constitué par un hydrogène, un halogène, un alkyle en C₁ à C₈, un halogénoalkyle en C₁ à C₈, un thioalcoxy en C₁ à C₈ et un alcoxy en C₁ à C₈ ; et
l'un de A₁ et A₂ est CH, et l'autre est N.

2. Médicament pour son utilisation selon la revendication 1, dans lequel chacun de R₁ et R₂ est indépendamment choisi dans le groupe constitué par un hydrogène, un halogène et un alkyle en C₁ à C₈.

3. Médicament pour son utilisation selon la revendication 1, dans lequel le composé carbamate de formule 1 est l'ester (R)-1-(2-chlorophényl)-2-tétrazol-2-yl-éthylique d'acide carbamique de formule 2 suivante :

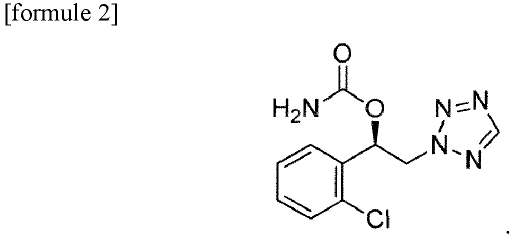

4. Combinaison pour son utilisation dans la prévention, le soulagement ou le traitement d'un trouble de stress aigu ou d'un trouble de stress post-traumatique, comprenant :
(a) une quantité thérapeutiquement efficace d'un composé carbamate de formule 1 qui suit, ou d'un sel, solvate ou hydrate pharmaceutiquement acceptable de celui-ci ; et
(b) un inhibiteur sélectif de la recapture de la sérotoninergique (SSRI) : 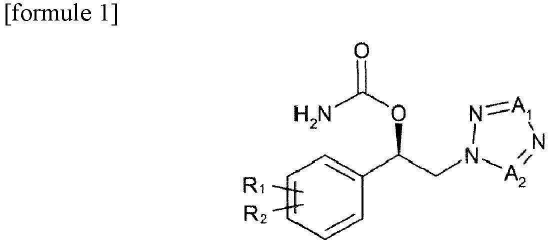 dans laquelle
chacun de R₁ et R₂ est indépendamment choisi dans le groupe constitué par un hydrogène, un halogène, un alkyle en C₁ à C₈, un halogénoalkyle en C₁ à C₈, un thioalcoxy en C₁ à C₈ et un alcoxy en C₁ à C₈ ; et
l'un de A₁ et A₂ est CH, et l'autre est N.

5. Combinaison pour son utilisation selon la revendication 4, dans laquelle l'inhibiteur sélectif de la recapture de la sérotoninergique est un ou plusieurs choisis dans le groupe constitué par la fluoxétine, la sertraline, la paroxétine et leurs sels pharmaceutiquement acceptables.

6. Combinaison pour son utilisation selon la revendication 4, dans laquelle le rapport en poids a/b est de 1000/1 à 1/1000.

7. Combinaison pour son utilisation selon la revendication 6, dans laquelle le rapport en poids a/b est de 100/1 à 1/100.

8. Combinaison pour son utilisation selon la revendication 4, qui comprend le composé de formule 1 en une quantité, basée sur la forme libre, de 12,5 mg à 500 mg.

9. Combinaison pour son utilisation selon la revendication 5, dans laquelle l'inhibiteur sélectif de la recapture de la sérotoninergique est la fluoxétine ou un sel pharmaceutiquement acceptable de celle-ci.

10. Combinaison pour son utilisation selon la revendication 9, dans laquelle l'inhibiteur sélectif de la recapture de la sérotoninergique est le chlorhydrate de fluoxétine.

11. Combinaison pour son utilisation selon la revendication 9, qui comprend de la fluoxétine en une quantité, basée sur la forme libre, de 10 à 60 mg.

12. Médicament pour son utilisation dans la prévention, le soulagement ou le traitement des symptômes d'un trouble de stress post-traumatique, comprenant une quantité thérapeutiquement efficace d'un composé carbamate de formule 1 qui suit, ou d'un sel, solvate ou hydrate pharmaceutiquement acceptable de celui-ci, et en outre un ou plusieurs véhicules pharmaceutiquement acceptables : 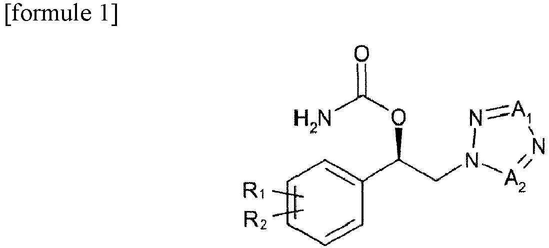 dans lequel
chacun de R₁ et R₂ est indépendamment choisi dans le groupe constitué par un hydrogène, un halogène, un alkyle en C₁ à C₈, un halogénoalkyle en C₁ à C₈, un thioalcoxy en C₁ à C₈ et un alcoxy en C₁ à C₈ ; et
l'un de A₁ et A₂ est CH, et l'autre est N.

13. Médicament pour son utilisation selon la revendication 12, dans lequel le symptôme de trouble de stress post-traumatique est un ou plusieurs choisis dans le groupe constitué par une amnésie complète ou partielle de l'événement traumatique, une récurrence chez un patient revivant l'événement traumatique ou l'évitement d'un stimulus associé à l'événement traumatique, des cauchemars, une irritabilité, une hyperexcitation, une hypervigilance, une rage, une médiocre concentration, et un retrait émotionnel.
